Europäisches Patentamt

European Patent Office

Office européen des brevets

(11) Publication number: **0 135 278**
**B1**

# EUROPEAN PATENT SPECIFICATION

(45) Date of publication of patent specification: **22.11.90**

(51) Int. Cl.⁵: **C 12 N 15/00, C 12 N 5/00**

(21) Application number: **84304751.5**

(22) Date of filing: **11.07.84**

(54) **Plasmid vector for mammalian cells.**

(30) Priority: **12.07.83 JP 125564/83**

(43) Date of publication of application:
**27.03.85 Bulletin 85/13**

(45) Publication of the grant of the patent:
**22.11.90 Bulletin 90/47**

(84) Designated Contracting States:
**AT BE CH DE FR GB IT LI LU NL SE**

(56) References cited:
**EP-A-0 054 223**

**NATURE, vol. 279, 28th June 1979, pages
811-816, Macmillan Journals, Ltd.; M. FRIED et
al.: "Infectivity in mouse fibroblasts of polyoma
DNA integrated into plasmid pBR322 or
lambdoid phage DNA"**

**CHEMICAL ABSTRACTS, vol. 97, no. 9, 30th
August 1982, page 153, abstract no. 67175g,
Columbus, Ohio, US; M. FRIED et al.: "Use of a
polyoma virus vector"**

(73) Proprietor: **TAISHO PHARMACEUTICAL CO.
LTD
24-1 Takata 3-chome Toshima-ku
Tokyo 171 (JP)**

(72) Inventor: **Oishi, Michio
29-12-1007, Hongo-5-chome
Bunkyo-ku Tokyo (JP)**
Inventor: **Uehara, Hiroshi
975, Nakamura
Ueda-shi (JP)**

(74) Representative: **Ablewhite, Alan James et al
MARKS & CLERK 57/60 Lincoln's Inn Fields
London WC2A 3LS (GB)**

(56) References cited:
**CHEMICAL ABSTRACTS, vol. 98, no. 5, 31st
January 1983, page 185, abstract no. 28884p,
Columbus, Ohio, US; G. RAUTMANN et al.:
"Complementation of a tsa mutant and
replication of a recombinant DNA carrying the
viral ori region in mouse cells transformed by
polyoma virus," VIROLOGY 1982, 122(2), 306-17**

Courier Press, Leamington Spa, England.

# EP 0 135 278 B1

**Description**

### Background of the Invention

#### 1. Field of Invention

This invention concerns novel vectors for mammalian cells which are isolated from mammalian cells.

In more detail, the invention concerns vectors which can form composite DNA with vectors specific to prokaryotic and yeast cells, can multiply in these cells as well as in mammalian cells, can multiply extrachromosomally in mammalian cells, can allow expression of desired gene(s) integrated in the vectors in mamalian cells and can be used as shuttle vectors.

#### 2. Description of the Prior Art

When attempts are made to produce useful materials by cloned genes in bacteria such as *Escherichia coli,* in most of the cases one encounters difficulties in producing the mammalian gene products due to the lack of the correct modification systems, for example for glycosylation and/or export in bacteria. Because of this, it is natural to attempt to produce useful materials in mammalian cells. However, at the present moment, no such useful vectors like pBR322 for *E. coli* are available for mammalian cells. Several potentially useful vectors for mammalian cells are following; (1) DNA from oncogenic viruses such as SV40 or polyoma. (2) Papilloma virus DNA. In case of (1), these DNA either integrate into host chromosomes or kill the host cells. In case of (2), sometimes there is no expression of integrated genes. Furthermore, the copy number of the exogenous DNA is only 50—100 copy/cell. These characteristics limit the use of these DNA for large scale production of useful materials. The plasmid vectors in this invention have eliminated these shortcomings of the vectors described above.

### Summary of the Invention

It is therefore an object of this invention to provide useful novel vectors which eliminate the shortcomings inherent in the currently available vectors and represent following characteristics. (1) they do not integrate into host chromosomes, (2) they have a high copy number per cell and (3) desired gene(s) integrated in the vectors can express its function while the vectors exist and replicate extrachromosomally in the host cells.

Other objects and advantages of this invention will be apparent from the following descriptions.

### Brief Description of the Drawings

Figure 1 shows restriction maps of plasmid LFI and LFII. Numbers within the circular maps express molecular size of the fragments between two adjacent cutting sites. Units are expressed as kilobases.

Figure 2 shows restriction maps of pdLFI and pdLFII.

Figure 3 shows restriction maps of cloned composite plasmids, pdLFI$_1$ (*tk*) and pdLFII$_1$ (*tk*).

Figure 4 shows restriction maps of cloned composite plasmids, pdLFI$_2$ (*tk*) and pdLFII$_2$ (*tk*).

Figure 5 shows restriction maps of cloned composite plasmids pdLFI$_3$ (*tk*) and pdLFII$_3$ (*tk*).

Figure 6 shows restriction maps of composite plasmids, pdLFI$_3$a (*tk*) and pdLFI$_3$b (*tk*).

Numbers in the circles express molecular size (bases) of Bam HI fragments of HSV-*tk* gene.

### Detailed Description of the Invention

The vectors of this invention are novel vectors named LFI and LFII and can be obtained from a mouse cell line which can be cultured *in vitro.* For example, a cell line B82—2, which was derived from mouse L cells, is grown in MEM (Eagle's Minimum Essential Medium) and after lysis of the cells with SDS (sodium dodecyl sulfate), the vectors are isolated and purified by centrifugation. The cell line B82—2 was isolated from one of mouse L cells (B82), as a cell line which has a high copy number of the concerned vectors. B82 cells were cultured in MEM with 10% calf serum and approximately each 50 cells were plated on total 5 plates with 90 mm diameter. After incubation for two weeks at 37°C, each colony which consisted of 500—1000 cells was examined by the procedure as will be described in Example 1-(4) and a cell was selected which had the highest copy number of vectors.

The cell line B82 is a useful tool in the recombinant DNA research and was first described by Littlefield (BBA., *95*, 14—22, 1965 and Experimental Cell Research *41*, 190—196, 1966); can be freely obtained from many research laboratories in the world (e.g., Mass. Cell Culture Centers at Massachusetts Institute of Technology, Harvard Medical School, University of Alabama, the mammalian cell stocks of Division of Enzymology, Institute of Applied Microbiology, University of Tokyo, etc.).

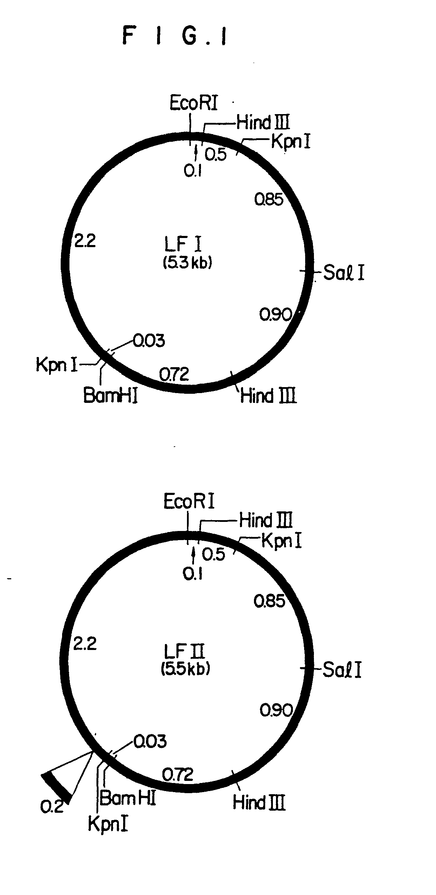

Molecular size of the vectors, LFI and LFII, were calculated to be approximately 5.3 kb and 5.5 kb, respectively, by analysis on agarose gel electrophoresis. When the restriction maps of the vectors obtained by using a variety of restriction enzymes were compared with that of polyoma virus DNA, it was found that both LFI and LFII consist of two kinds of DNA structures, one is present in polyoma virus DNA and the other is not present in polyoma virus DNA. LFII has an extra DNA structure of 0.2 kb which is inserted at approximately 500 bp clockwise from a Kpn I site (near Bam HI site) (see Figure 1) in addition to LFI structure. Restriction maps of LFI and LFII using restriction enzymes, Eco RI, Sal I, Bam HI, Hind III, Hpa II and Kpn I were compared with the restriction map of polyoma virus DNA with the same enzymes. LFI and polyoma virus DNA are cut approximately at the same sites where Eco RI, Bam Hi, Hind III and Kpn I cut. On the other hand, LFI possesses a cutting site by Sal I which does not exist in polyoma DNA. Total DNA base pairs of LFI are almost equal to those of polyoma virus DNA. LFII also possesses a cutting site by Sal I which does not exist in polyoma virus DNA but it also has cutting sites by Eco Ri, Bam HI, Hind III, Kpn I which are located at almost the same places as located in polyoma virus DNA. The total base pairs of LFII (5.5 kb) are roughly equal to those of polyoma virus DNA (5.292 kb). While polyoma DNA is cut at eight sites by Hpa II, LFI and LFII have 11 and 14 cutting sites by Hpa II, respectively. These results indicate that LFI and LFII differ from polyoma virus DNA in at least three base sequences.

When we calculated copy numbers of the concerned vectors in the cell based upon the results obtained by agarose gel electrophoresis, the maximum copy number of LFI per cell is approximately 5,000 copies and that of LFII is approximately 1,000 copies. The number of polyoma virus genome per cell is only 10 to 50 copies.

We have demonstrated that the concerned vectors exist extrachromosomally without being integrated in B82—2 chromosomes. This was done by Southern hybridization (E. M. Southern; J. Mol. Biol. *98*, 503, 1975) of the total DNA isolated from cultured mouse L cell B82—2 after having treated the DNA by a restriction enzyme, Eco RI or Sal I.

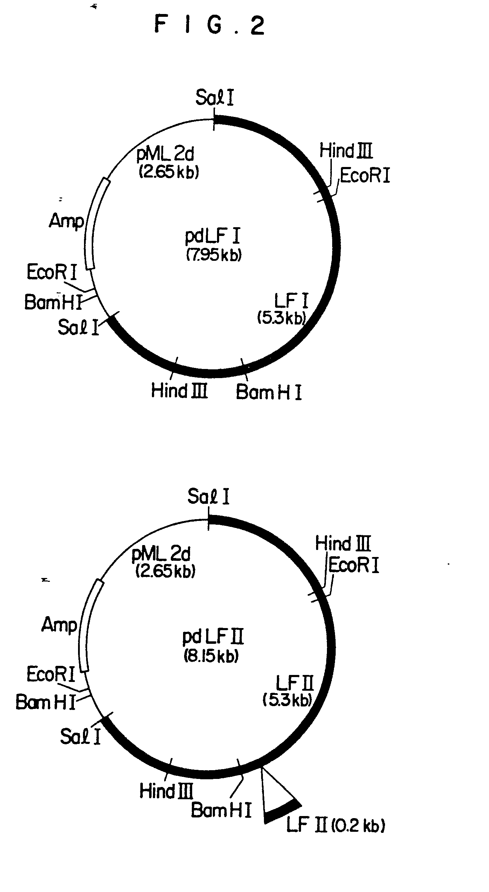

The concerned vectors LFI and LFII can form composite plasmids with bacterial plasmids. For example they can form composite plasmids with a derivative of pBR322 DNA, pML2d, and the composite plasmids can propagate in *E. coli*. This was demonstrated by the following experiments. First, 100 ng of LFI and LFII digested with Sal I and 20 ng of pML2d (2.65 kb) DNA digested likewise were mixed and ligated in the presence of 0.2 units of T4 DNA ligase and 2.5 mM ATP. When *E. coli* LE392 was transformed by the DNA by $CaCl_2$ procedure (T. Maniatis, E. F. Fritsch, J. Sambrook; Molecular Cloning, p250, Cold Spring Harbour Lab., 1982), ampicillin (20 µg/ml) resistant transformants ($Amp^r$) were obtained. DNAs were then prepared from these $Amp^r$ cells and they were hybridized with $^{32}p$-labeled LFI and LFII. Presence of 7.95 kb and 8.15 kb circular DNA in $Amp^r$ cells was further demonstrated by agarose gel electrophoresis. These composite plasmids were named as pdLFI (7.95 kb) and pdLFII (8.15 kb), respectively (Figure 2).

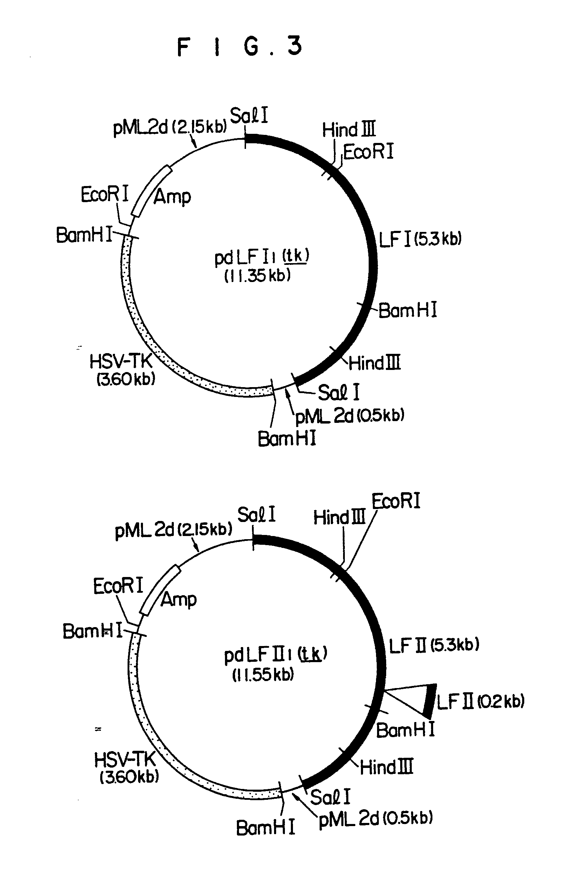

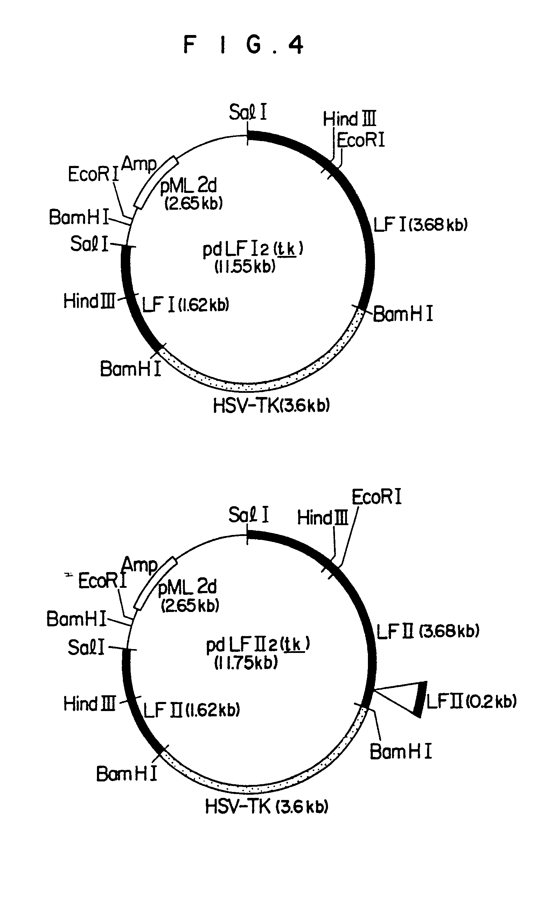

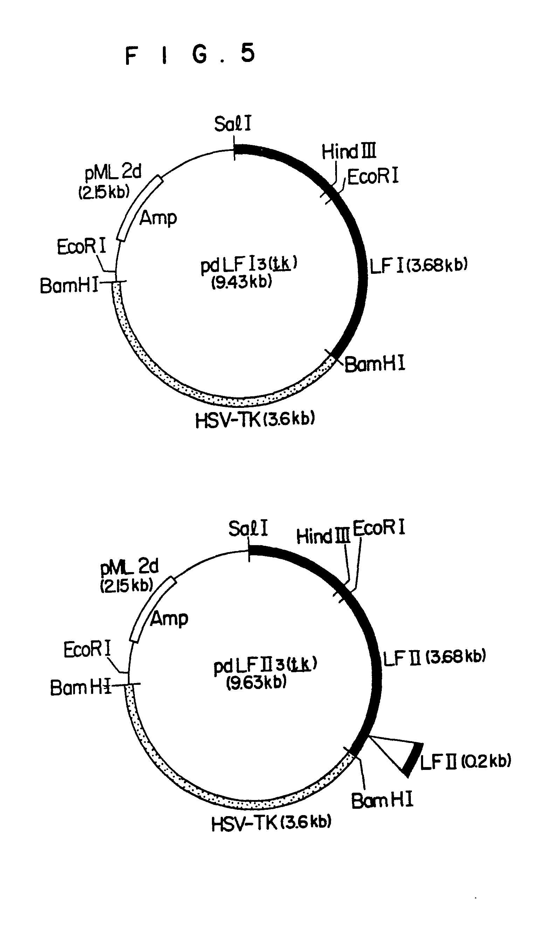

Furthermore, the concerned vectors, after integration of a gene(s) for useful materials into them, still can exist as extrachromosomal plasmids in mammalian cells, can replicate autonormously and can express the integrated gene(s). The above described composite plasmids pdLFI and pdLFII, which were obtained from LFI and LFII and a derivative of pBR322, pML2d, were cut by a restriction enzyme Bam HI and ligated with herpes simplex virus thymidine kinase gene (HSV *tk*) which were cut by Bam HI. *E. coli* (LE392) was then transformed with the resulting DNA and from $Amp^r$ transformants six different plasmids were obtained (Figures 3, 4, 5). These plasmids were named as $pdLFI_1(tk)$, $pdLFI_2(tk)$, $pdLFI_3(tk)$, $pdLFII_1(tk)$, $pdLFII_2(tk)$ and $pdLFII_3(tk)$, respectively. Among them, molecular size of $pdLFI_3(tk)$ and $pdLFII_3(tk)$ are approximately 9.45 and 9.65 kb, respectively and a portion of the original DNA sequences of pdLFI and pdLFII, approximately 2.10 kb, is deleted in these vectors. When mouse L cells defective in thymidine kinase were transfected with $pdLFII_3(tk)$ DNA by the calcium phosphate method and incubated in HAT medium (W. H. Lewis, P. R. Srinivason, N. Stock, L. Siminovitch; Somatic Cell Genetics *6*, 333, 1980), colonies appeared after 2 or 3 weeks. The $Tk^+$ colonies were then recultured, the cells were resuspended in TE buffer (10 mM Tris-HCl, pH 7.4, 10 mM EDTA) and lysed with SDS. The total DNA purified from the lysates by centrifugation were treated with a restriction enzyme, either Eco RI or Sal I or Xho I, electrophoresed on agarose gel and hybridized (Southern hybridization) with $^{32}p$-labeled $pdLFII_3(tk)$ DNA as a probe. Since hybridized band after autoradiography coincided with the position where $pdLFII_3(tk)$ DNA migrates, it has become evident that all the $pdLFII_3(tk)$ DNA exists extrachromosomally in $Tk^+$ mouse cells. It was demonstrated from these results that while $pdLFII_3(tk)$ DNA replicates extrachromosomally as plasmids in L cells, the integrated gene (*tk*) expresses its function. When *E. coli* was transformed with DNA prepared from the $Tk^+$ cells, $Amp^r$ colonies appeared. DNA isolated from the $Amp^r$ colonies (D. S. Holmes, M. Quigley; Anal Biochem *114*, 193, 1981) was subjected to agarose gel electrophoresis. A DNA band was detected at the position where $pdLFII_3(tk)$ DNA migrates. These results indicate that $pdLFII_3(tk)$ DNA can be used as a shuttle vector for *E. coli* to L cells to *E. coli*, while maintaining its original DNA structure and expressing integrated genes (*amp* in *E. coli* and *tk* in L cells). Similar results were obtained with $pdLFI_1(tk)$, $pdLFI_2(tk)$, $pdLFI_3(tk)$, $pdLFII_1(tk)$ and $pdLFII_2(tk)$.

As described above, the concerned vectors LFI and LFII are novel vectors, which can form composite plasmids with bacterial plasmids such as pBR322 (pML2d), can propagate extrachromosomally in bacteria and mammalian cells, can allow the expression of a useful gene(s) integrated in the vectors and have a high copy number in the cells. Also, it is clear from the relationships between $pdLFII_1$, $pdLFII_2$ and $pdLFII_3$, or between $pdLFI_1$, $pdLFI_2$ and $pdLFI_3$ that functions which enable LFI and LFII to replicate autonormously reside in a part of LFI and LFII structure as in other plasmids. From these facts, it is easily deduced that derivatives of LFI and LFII with another DNA possess functions similar to those of LFI and LFII. Therefore, it is obvious that usefulness of LFI and LFII should not be limited to themselves but can be extended to the DNA which is obtained by modification of LFI and LFII.

## Example 1

### (1) *Preparation of vector LFI and LFII.*

Cell line B82 (from the cell collection, Division of Enzymology, Institute of Applied Microbiology, University of Tokyo) was cultured in MEM with 10% calf serum and approximately each 50 cells were

3

plated on total 5 plates of 90 mm diameter made of plastics. After incubation for two weeks at 37°C, each colony which consisted of 500—1000 cells was examined by the procedure as will be described in Example 1-(4) mentioned later and a cell line having the highest copy number of the vectors of this invention was selected as cell line B82—2. B82—2 cells were cultured in MEM containing 10% calf serum for 5 days at 37°C. Approximately $2 \times 10^7$ cells were resuspended in 1 ml TE buffer (10 mM Tris-HCl, pH 7.4, 10 mM EDTA) and lysed with SDS (final concentration 0.6%). To the lysate, NaCl (final 1.0 M) was added and after leaving for 8 hrs at 4°C, the sample was centrifuged (15,000 rpm 30 min). The supernatant fraction was mixed with an equal volume of phenol-chloroform mixture (1:1), shaken and centrifuged. The aqueous phase was then mixed with chloroform-isopropylalcohol (24:1) and DNA was precipitated with ethanol from the aqueous phase after centrifugation. The DNA precipitate was dissolved in TE buffer and electrophoresed on agarose gel with 0.5 µg/ml of ethidiumbromide in Tris-borate-EDTA buffer (89 mM Tris-borate, pH 7.8, 89 mM boric acid, 2 mM EDTA) for 4 hrs at 3 volts/cm. After confirming two distinct bands under a UV lamp, the bands were transferred to a DEAE membrane filter paper by electrophoresis for 1 hr at 3 volts/cm. The filter was then washed with TES buffer (10 mM Tris-HCl, pH 8.0, 1 mM EDTA, 0.3 M NaCl) and DNA was eluted with a TES elution buffer (10 mM Tris-HCl, pH 8.0, 1 M NaCl) for 2 hrs at 55°C. The DNA was recovered as a precipitate from the eluate by adding 2.5 vols of 99.5% ethanol. The DNA thus obtained was dissolved in TE buffer (10 mM Tris-HCl pH 8.0, 1 mM EDTA) and kept at 4°C. DNAs with smaller molecular weight (faster migrating DNA) and with larger molecular weight (slower migrating DNA) were named as LFI and LFII, respectively.

(2) *Determination of the molecular size.*

The DNA fraction (2 µg) obtained in (1) was treated with a restriction enzyme, Sal I (15 units) for 5 hrs at 37°C in a buffer containing 10 mM Tris-HCl, pH 7.5, 7 mM MgCl$_2$, 5 mM dithiothreitol (DTT) and 50 mM NaCl. After electrophoresis as described in (1), two bands, as previously observed in (1), were detected but at different locations. The molecular size of the DNA was detemined to be approximately 5.3 kb and 5.5 kb after comparison with the positions of standard λ DNA which was digested with Hind III and electrophoresed under the same condition. From these experiments, it became clear that B82—2 cells contain two circular DNA with 5.3 kb (LFI) and 5.5 kb (LFII). Furthermore, 5.3 kb DNA was several fold more abundantly present than 5.5 kb DNA.

(3) *Construction of the restriction maps.*

200 ng of LFI and LFII DNA was treated with a restriction enzyme (10 units per 1 µg DNA) including Eco RI, Bam HI, Hind III, Sal I, Kpn I, Bgl II, Xho I, and Hpa II and electrophoresed as described in (1). From the migration patterns of DNA, it has become clear that LFI and LFII have a common DNA structure and LFII has an extra DNA structure of 0.2 kb in addition to LFI DNA which is inserted at approximately 500 bp clockwise from a Kpn I site (near Bam HI site). Molecular sizes of DNA obtained by treatment with a variety of restriction enzymes are shown in Table 1 (LFI) and Table 2 (LFII).

Based upon the cutting site by Eco RI (at 0), distances of cutting sites produced by the restriction enzymes (Bam HI, Hind III, Kpn I, Sal I) are shown in the following figure (Figure 1) and Table 3.

From the results of Tables 1, 2 and 3, restriction maps for LFI and LFII were constructed and they are shown in Figure 1.

## TABLE 1

| Restriction enzymes | Number of Cutting sites | Molecular size (kb) | |
|---|---|---|---|
| | | a* | b* |
| Eco RI | 1 | 5.3 | |
| Bam HI | 1 | 5.3 | |
| Hind III | 2 | 3.04 | 2.26 |
| Sal I | 1 | 5.3 | |
| Kpn I | 2 | 2.8 | 2.5 |
| Bgl II | 0 | | |
| Hpa II | 10 | | |
| Xho I | 0 | | |

Note: * *a* and *b* represent fragments, respectively.

TABLE 2

| Restriction enzymes | Number of Cutting sites | Molecular size (kb) | |
|---|---|---|---|
| | | a* | b* |
| Eco RI | 1 | 5.5 | |
| Bam HI | 1 | 5.5 | |
| Hind III | 2 | 3.24 | 2.26 |
| Sal I | 1 | 5.5 | |
| Kpn I | 2 | 3.0 | 2.5 |
| Bgl II | 0 | | |
| Hpa II | 14 | | |
| Xho I | 0 | | |

Note: * a and b represent fragments, respectively.

TABLE 3

| Restriction enzymes | Positions from Eco RI cutting site (kb) | |
|---|---|---|
| Eco RI | 0 | |
| Bam HI | 3.07 | |
| Hind III | 0.1 | 2.35 |
| Kpn I | 0.6 | 3.1 |
| Sal I | 1.45 | |
| Bgl II | No cutting site | |
| Xho I | No cutting site | |

(4) *Determination of the copy number.*

From approximately $2 \times 10^7$ cells cultured as described in (1), DNA was extracted as described in (1), electrophoresed on agarose gels with different amounts of SV40 DNA with molecular size of 5.2 kb as described in (2). The intensities of the ethidiumbromide-DNA revealed by UV light were compared with those of 5.2 kb SV40 DNA. From the number of cells ($2 \times 10^7$) used, the number of LFI and LFII per cell was calculated to be approximately 5,000 copies and 1,000 copies, respectively.

(5) *Evidence for the extrachromosomal existence of the vectors*

B82—2 cells cultured as described in (1) were lysed with SDS and total DNA was extracted with phenol-chloroform (1:1). The DNA was purified by the procedure described in (1) and separted into four fractions. After treating them with no enzyme (i), with Eco RI (ii), with Sal I (iii) and with Xho I (iv), they were electrophoresed on agarose gel as described in (1). After alkaline denaturation (T. Maniatis, E. F. Fritsch, J. Sambrook; Molecular Cloning, P 383, C.S.H.L., 1982) and transfer to a nitrocellulose filter, DNA was then hybridized (Southern hybridization) with LFI and LFII as probes which have been labeled with $^{32}$P by nick translation using DNA polymerase I. The DNA bands which were hybridized with the probes were examined by radioautography. The hybridized DNA bands were detected at positions corresponding to 5.3 kb and 5.5 kb with (ii) and (iii) samples. The hybridized DNA bands with (i) and (iv) samples were located at the same position. No other band was detected. From these experiments, it was concluded that in B82—2 cells LFI and LFII exist and replicate extrachromosomally without being integrated in the chromosome.

Example 2

Construction of composite plasmids with *E. coli* plasmid pBR322 (pML2d) (shuttle vectors) which can replicate in *E. coli*

(a) Composite plasmid with LFI and pML2d.

Under the condition described in Example 1-(2), LFI (100 ng) and pML2d DNA (20 ng) which had been cut by Sal I were mixed, heated at 65°C for 10 min, rapidly cooled, mixed with 2.5 vols of ethanol in the presence of 0.3 M sodium acetate and left for at least 2 hrs at −20°C. The samples were centrifuged at 15,000 rpm for 20 min and the precipitate was dried *in vacuo*. To the precipitate, distilled water (7.5 μl), concentrated (× 10) ligation buffer (1 μl, 660 mM Tris-HCl, pH 7.5, 66 mM MgCl$_2$) and calf serum albumin solution (0.1 μl, 2 mg/40 μl) were added. After incubation at 42°C for one hr, ATP (0.25 μl of 0.1 M), DTT (1 μl of 0.1 M) and T4 DNA ligase (0.15 μl of 1.5 units per μl) were added and the samples were left for two days at 4°C. Seventy μl of Tris buffer (10 mM Tris-HCl, pH 8.0, 1 mM EDTA), was then added and after electrophoresing a portion of the samples to confirm appearance of new bands, the DNA was used for bacterial transformation using *E. coli* (LE392) as a recipient by CaCl$_2$ method (T. Maniatis, E. F. Fritsch, J. Sambrook; Molecular Cloning p250, Cold Spring Harbour Lab. 1982). Resultant amplicillin resistant (20 μg/ml) colonies were replicated on nitrocellulose filter papers and the papers were placed for 7 min (on Whatman 3MM papers) in 10 ml of 0.5 M NaOH — 1.5 M NaCl solution to denature DNA. The nitrocellulose papers were then neutralyzed by soaking them in Tris buffer (1 M Tris-HCl, pH 6.7, 1.5 M NaCl) for 7 min, followed by soaking them in × 2 SSC which was prepared by 10 fold dilution of × 20 SSC (3 M NaCl, 0.3 M Na$_3$-citrate, pH 7.0). After drying in air for 30 min, the papers were heated for 2 hrs at 80°C in an oven. The papers were then soaked in prehybridization solution (1:1 mixture of × 5 SSC and × 4 Denhardt solution which consists of 5 times diluted × 20 Denhardt solution; 0.4% Ficoll, 0.4% calf serum albumin, 0.4% polyvinylpyrrolidone), and after incubation for 2 hrs at 70°C, the papers were dried in air. They were then placed and incubated at 65°C for overnight in plastic bags with a mixture of solution (100 μl) consisting of 15 μl of $^{32}$P-labeled LFI DNA (2 × 10$^5$ cpm/ml, prepared by nick-translation, 3 μl of salmon sperm DNA (10 mg/ml) and 82 μl of H$_2$O, which had been heated at 100°C for 10 min and quickly cooled, and the prehybridization solution (900 μl) described above. The papers were then washed three times with × 2 SSC solution at 55°C for 20 min. After having dried in air, hybridization of the probes ($^{32}$P-LFI DNA) with the DNA from bacteria was confirmed by radioautography (colony hybridization). The Amp$^r$ cells thus confirmed were then grown and lysed with lysozyme and SDS. Total DNA was then extracted by the procedures described in Example 1-(5), treated with restriction enzymes (Sal I, Eco RI, Bam HI) and electrophoresed on agarose. The presence of circular DNA with 7.95 kb in Amp$^r$ cells was then demonstrated by Southern hybridization using $^{32}$P-labeled LFI DNA as a probe.

(b) Composite plasmid with LFII and pML2d.

The presence of circular DNA with 8.15 kb in Amp$^r$ cells were also confirmed by the same experiments as described in (a) except that LFII DNA was used instead of LFI DNA. The composite plasmids obtained in (a) and those obtained in (b) were named as pdLFI and pdLFII, respectively.

Example 3

Usefulness of pdLFI and pdLFII as shuttle vectors and evidence for extrachromosomal gene expression of a cloned gene in mammalian cells

The composite plasmid (pdLFI and pdLFII) obtained from LFI (and LFII) and pBR322 (pML2d) as described in Example 2 was cut by a restriction enzyme Bam HI. Twenty ng of the DNA was then mixed with 100 ng of a Bam HI fragment of herpes simplex virus thymidine kinase gene derived from pFG5 (Proc. Natl. Acad. Sci. USA *76*, 3755, 1979) and ligated by the procedures described in Example 2. *E. coli* (LE392) was then transformed with the DNA and Amp$^r$ transformants were subjected to colony hybridization as described in Example 1-(5) using $^{32}$P-labeled HSV-*tk* as a probe. The presence of bacterial DNA which was hybridized with $^{32}$P-HSV-*tk* DNA was confirmed by radioautography. Furthermore, DNA isolated from the Amp$^r$ transformants according to the procedures described in Example 1-(1) was analyzed by various restriction enzymes (Sal I, Bam HI, Eco RI, Hind III) as described in Example 1-(3). Six different plasmids were then obtained from Amp$^r$ transformants and they were named as pdLFI$_1$(*tk*), pdLFI$_2$(*tk*), pdLFI$_3$(*tk*), pdLFII$_1$(*tk*), pdLFII$_2$(*tk*) and pdLFII$_3$(*tk*) (Figures 3, 4 and 5). Among them, molecular size of pdLFI$_3$(*tk*) and pdLFII$_3$(*tk*) are approximately 9.43 kb and 9.63 kb, respectively. The smaller sizes of these DNA were resulted by a deletion of a portion (approximately 2.12 kb) from plasmids pdLFI and pdLFII, respectively.

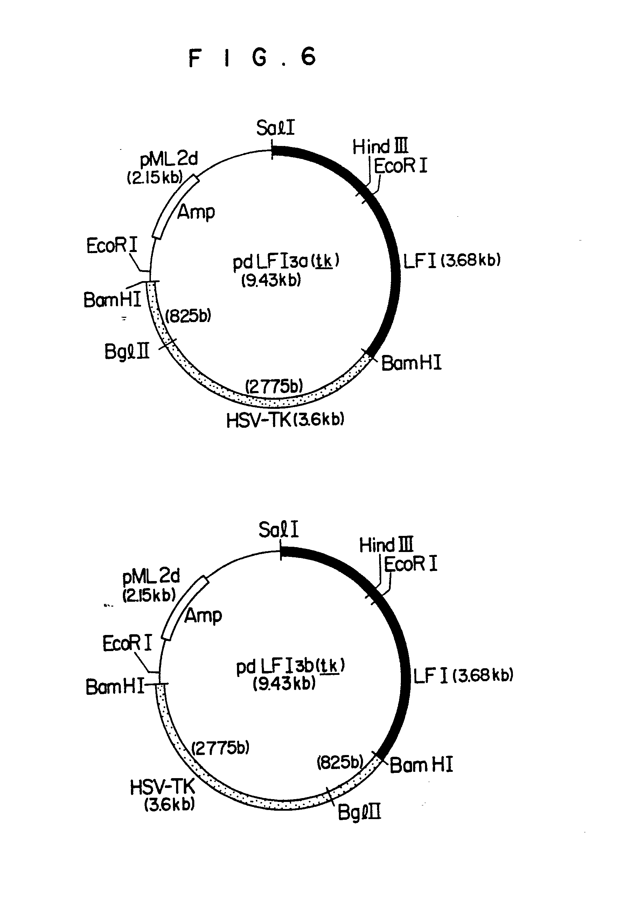

Analyses of pdLFI$_3$(*tk*) by restriction enzymes Sal I and Bgl II according to the procedures described in Example 1-(5) having revealed that two kinds of forms exist in pdLFI$_3$(*tk*). They are (a), which produced approximately 3.1 kb and 6.4 kb fragments upon digestion with Sal I, followed by Bgl II and (b), which produced approximately 4.4 kb and 5.0 kb fragments by the same treatment. In combining these results and an observation that the restriction enzyme Bgl II cuts HSV-*tk* gene obtained by Bam HI treatment into two fragments with 825 bp and 2,775 bp, it was concluded that a composite plasmid ligated with HSV-*tk* gene in the opposite direction also exists (Figure 6). Naturally this situation should be applicable to pdLFI$_1$(*tk*), pdLFI$_2$(*tk*), pdLFII$_1$(*tk*), pdLFII$_2$(*tk*) and pdLFII$_3$(*tk*). Also it should be applicable to composite plasmids of pdLFI and pdLFII, which were constructed from LFI (and LFII) and pML2d.

When mouse L cells deficient in thymidine kinase (*tk*) were treated with pdLFII$_3$(*tk*) DNA-calcium phosphate (W. H. Lewis, P. R. Srinivason, N. Stock, L. Siminovitch; Somatic Cell Genetics *6*, 333, 1980) and incubated in HAT medium, colonies were formed after two weeks. The Tk$^+$ colonies were then recultured and the total DNA was prepared by the procedure described in Example 1-(1) and Example 1-(5). After electrophoresis on agarose gel, Southern hybridization was performed using $^{32}$P-labeled pdLFII$_3$(*tk*) DNA as a probe. Results of autoradiography showed that the hybridized band was located at the position where pdLFII$_3$(*tk*) migrated, indicating that all of the pdLFII$_3$(*tk*) DNA locate extrachromosomally in mouse L cells.

Furthermore, when DNA prepared from the Tk$^+$ cells according to the procedure described in Example 1-(1) was used as a donor for the transformation of *E. coli* (LE392) as described in Example 2, Amp$^r$ colonies appeared. DNA was prepared from the Amp$^r$ cells (D. S. Holmes, M. Quigley; Anal Biochem. *114*, 193, 1981) and subjected to electrophoresis on agarose gel. A band corresponding to the molecular size of pdLFII$_3$(*tk*) was observed. From these results, it was demonstrated that pdLFII$_3$(*tk*) plasmid can express its genes (Amp in *E. coli*, *tk* in L cells), while maintaining its original structure during transfers from E. coli-L cells-*E. coli* and can be used as a shuttle vector (mammalian cells — *E. coli*). Similar results were obtained with pdLFI$_1$(*tk*), pdLFI$_2$(*tk*), pdLFI$_3$(*tk*), pdLFII$_1$(*tk*) and pdLFII$_2$(*tk*).

From the experiments described above, it was proved that plasmids LFI and LFII can form composite plasmids with bacterial plasmids such as pBR322 (pML2d). Furthermore, after integration of a gene(s) responsible for the production of useful materials, they can still replicate extrachromosomally and the integrated genes can be expressed in mammalian cells.

**Claims**

1. A plasmid vector capable of extrachromosomal replication in eucaryotic host cells which possesses restriction enzyme cutting sites at:

0.10 kb and 2.35 kb by Hind III,
0.60 kb and 3.10 kb by Kpn I,
3.07 kb by Bam HI, and,
1.45 kb by Sal I,

the said cutting sites being based on an Eco RI cutting site at 0.00 kb prepared by:

1) culturing mouse L cell line B82 as defined herein.
2) lysing the cells, and
3) isolating the plasmid vectors.

2. A plasmid vector LFI as claimed in claim 1 with a molecular size of 5.3 kb.

3. A plasmid vector LFII as claimed in claim 1 with a molecular size of 5.5 kb.

4. A composite plasmid pdLFI comprising a vector as claimed in claim 2 and pML2d, as shown by the restriction map.

5. A composite plasmid pdLFII comprising a vector as claimed in claim 3 and pML2d as shown by the restriction map.

6. A composite plasmid pdLFI₁(*tk*) comprising a plasmid as claimed in claim 4 and the thymidine kinase gene from pFG5, as shown by the restriction map.

7. A composite plasmid pdLFII₁(*tk*) comprising a plasmid as claimed in claim 5 and the thymidine kinase gene from pFG5, as shown by the restriction map.

8

8. A composite plasmid pdLFI₂(*tk*) comprising a plasmid as claimed in claim 4 and the thymidine kinase gene from pFG5, as shown by the restriction map.

9. A composite plasmid pdLFII₂(*tk*) comprising a plasmid as claimed in claim 5 and the thymidine kinase gene from pFG5, as shown by the restriction map.

10. A composite plasmid pdLFI₃(*tk*) comprising a plasmid as claimed in claim 4 and the thymidine kinase gene from pFG5, as shown by the restriction map.

11. A composite plasmid pdLFII₃(*tk*) comprising a plasmid as claimed in claim 5 and the thymidine kinase gene from pFG5, as shown by the restriction map.

12. A composite plasmid pdLFI₃ₐ(*tk*) comprising a plasmid as claimed in claim 10 and the thymidine kinase gene from pFG5, as shown by the restriction map.

13. A composite plasmid pdLFI₃ᵦ(*tk*) comprising a plasmid as claimed in claim 10 and the thymidine kinase gene from pFG5, as shown by the restriction map.

10

**Patentansprüche**

1. Plasmidvektor mit der Fähigkeit zur extrachromosomalen Replikation in eukariontischen Wirtszellen, der Restriktionsenzymschnittstellen besitzt bei:

0,10 kb und 2,35 kb durch Hind III,

0,60 kb und 3,10 kb durch Kpn I,

3.07 kb durch Bam HI und

1,45 kb durch Sal I,

wobei die Schnittstellen ab einer Eco RI-Schnittstelle bei 0,00 kb gezählt werden und der Plasmidvektor hergestellt wird durch:

(1) Kultur der Maus-L-Zellinie B82, wie hierin definiert,

(2) Lyse der Zellen, und

(3) Isolierung des Plasmidvektors.

2. Plasmidvektor LFI nach Anspruch 1 mit einem Molekulargewicht von 5,3 kb.

3. Plasmidvektor LFII nach Anspruch 1 mit einem Molekulargewicht von 5,5 kb.

4. Zusammengesetztes Plasmid pdLFI, enthaltend einen Vektor gemäss Anspruch 2 und pML2d, wie in der Restriktionskarte gezeigt

5. Zusammengesetztes Plasmid pdLFII, enthaltend einen Vektor gemäss Anspruch 3 und pML2d, wie in der Restriktionskarte gezeigt

6. Zusammengesetztes Plasmid pdLFI$_1$(tk), enthaltend ein Plasmid gemäss Anspruch 4 und das Thymidinkinasegen von pFG5, wie in der Restriktionskarte gezeigt

11

7. Zusammengesetztes Plasmid pdLFII₁(*tk*), enthaltend ein Plasmid gemäss Anspruch 5 und das Thymidinkinasegen von pFG5, wie in der Restriktionskarte gezeigt

8. Zusammengesetztes Plasmid pdLFI₂(*tk*), enthaltend ein Plasmid gemäss Anspruch 4 und das Thymidinkinasegen von pFG5, wie in der Restriktionskarte gezeigt

12

**EP 0 135 278 B1**

9. Zusammengesetztes Plasmid pdLFII$_2$(*tk*), enthaltend ein Plasmid gemäss Anspruch 5 und das Thymidinkinasegen von pFG5, wie in der Restriktionskarte gezeigt

10. Zusammengesetztes Plasmid pdLFI$_3$(*tk*), enthaltend ein Plasmid gemäss Anspruch 4 und das Thymidinkinasegen von pFG5, wie in der Restriktionskarte gezeigt

11. Zusammengesetztes Plasmid pdLFII$_3$(*tk*), enthaltend ein Plasmid gemäss Anspruch 5 und das Thymidinkinasegen von pFG5, wie in der Restriktionskarte gezeigt

12. Zusammengesetztes Plasmid pdLFl₃ₐ(*tk*), enthaltend ein Plasmid gemäss Anspruch 10 und das Thymidinkinasegen von pFG5, wie in der Restriktionskarte gezeigt

13. Zusammengesetztes Plasmid pdLFl₃ᵦ(*tk*), enthaltend ein Plasmid gemäss Anspruch 10 und das Thymidinkinasegen von pFG5, wie in der Restriktionskarte gezeigt

**Revendications**

1. Vecteur plasmidique, capable de replication extrachromosomique dans des cellules-hôtes possédant des sites de compure par enzyme de restriction à:
0,10 kb et 2,35 par Hind III,
0,60 kb et 3,10 par Kpn I,
3,07 kb par Bam HI, et
1,45 kb par Sal I
lesdits sites de coupure étant basés sur un site dde coupure par Eco RI à 0,00 kb, préparé par:
1) culture de la souche B82 de cellules L de souris, telle que définie ci-inclus,
2) lyse des cellules, et,
3) isolation des vecteurs plasmidiques.

2. Vecteur plasmidique LFI, selon la revendication 1, ayant une taille moléculaire de 5,3 kb.

3. Vecteur plasmidique LFII, selon la revendication 1, ayant une taille moléculaire de 5,5 kb.

4. Plasmide composite pdLFI, comprenant un vecteur selon la revendication 2, et pML2d, comme représenté par le schéma de restriction

5. Plasmide composite pdLFII, comprenant un vecteur selon la revendication 3, et pML2d, comme représenté par le schéma de restriction

6. Plasmide composite pdLFI₁(*tk*), comprenant un plasmide selon la revendication 4 et le gène de thymidine kinase provenant de pFG5, comme représenté par le schéma de restriction

7. Plasmide composite pdLFII₁(*tk*), comprenant un plasmide selon la revendication 5 et le gène de thymidine kinase de pFG5, comme représenté par le schéma de restriction

pML 2d (2.15kb)  Sal I  Hind III  EcoRI
EcoRI  Amp
BamHI
pdLFIII (*tk*)  (11.55kb)  LF II (5.3kb)
LF II (0.2kb)
BamHI
HSV-TK (3.60kb)  Hind III
Sal I
BamHI  pML 2d (0.5kb)

8. Plasmide composite pdLFI₂(*tk*), comprenant un plasmide selon la revendication 4 et le gène de thymidine kinase provenant de pFG5, comme représenté par le schéma de restriction

Sal I  Hind III  EcoRI
EcoRI  Amp
pML 2d (2.65 kb)
BamHI
Sal I  pdLFI2 (*tk*) (11.55kb)  LF I (3.68 kb)
Hind III  LF I (1.62 kb)  BamHI
BamHI
HSV-TK (3.6 kb)

9. Plasmide composite pdLFII₂(*tk*), comprenant un plasmide selon la revendication 5 et le gène de thymidine kinase de pFG5, comme représenté par le schéma de restriction

EcoRI
Sal I  Hind III
EcoRI  Amp
pML 2d (2.65 kb)
BamHI
Sal I  pdLFII2 (*tk*) (11.75kb)  LF II (3.68kb)
LF II (0.2kb)
Hind III  LF II (1.62kb)  BamHI
BamHI
HSV-TK (3.6 kb)

16

10. Plasmide composite pdLFI₃(*tk*) comprenant un plasmide selon la revendication 4 et le gène de thymidine kinase de pFG5, comme représenté par le schéma de restriction

11. Plasmide composite pdLFII₃(*tk*) comprenant un plasmide selon la revendication 5 et le gène de thymidine kinase de pFG5, comme représenté par le schéma de restriction

12. Plasmide composite pdLFI₃ₐ(*tk*) comprenant un plasmide selon la revendication 10 et le gène de thymidine kinase de pFG5, comme représenté par le schéma de restriction

13. Plasmide composite pdLFI$_{3b}$(*tk*) comprenant un plasmide selon la revendication 10 et le gène de thymidine kinase de pFG5, comme représenté par le schéma de restriction

# F I G . I

# F I G . 2

# F I G . 3

# F I G . 4

# F I G . 5

# F I G . 6